# EUROPÄISCHE PATENTANMELDUNG

(11) **EP 0 965 576 A1**
(43) Veröffentlichungstag der Anmeldung: **22.12.1999**
(21) Anmeldenummer: 99108046.6
(22) Anmeldetag: 23.04.1999
(51) Int. Cl.: C07C 51/02, C07C 51/487, C07C 51/41

(54) **Verfahren zum Erzeugen von Weinsäure aus einem Kaliumhydrogentartrat enthaltenden Rohmaterial**

(30) Priorität: 04.05.1998 DE 19819884
(71) Anmelder: Metallgesellschaft Aktiengesellschaft, 60325 Frankfurt am Main (DE); Spezial Chemie Leuna GmbH & CO. KG, 06236 Leuna (DE)
(72) Erfinder: Bönsch, Rudolf Dr., 55299 Nackenheim (DE); Stein, Dieter, 65191 Wiesbaden (DE); Erb, Klaus Dr., 38640 Goslar (DE)
(74) Vertreter: Revesz, Veronika

(57) **Zusammenfassung**

Das Kaliumhydrogentartrat (KHT) enthaltende Rohmaterial wird in einer ersten Umsetzungsstufe mit wäßriger Kalilauge verrührt, wobei man KHT praktisch vollständig zu Dikaliumtartrat (DKT) umsetzt. Die DKT enthaltende wäßrige Lösung wird in einer Fällungsstufe mit zudosierter Säure bei einem pH-Wert von 2,0 bis 5,0 verrührt und eine auskristallisiertes KHT enthaltende Suspension erzeugt. Aus der Suspension trennt man auskristallisiertes KHT ab, wäscht es mit Wasser und erzeugt eine zu mindestens 80 Gew.-% mit KHT gesättigte Lösung. Aus dieser Lösung entfernt man Kalium, und man erhält eine wäßrige Weinsäure-Produktlösung, aus der man Wasser mindestens teilweise entfernt.

## Beschreibung

Die Erfindung betrifft ein Verfahren zum Erzeugen von Weinsäure aus einem Rohmaterial, dessen Trockensubstanz zu mindestens 5 Gew.-% aus Kaliumhydrogentartrat (KHT) besteht. Als Rohmaterial kann zum Beispiel Weinhefe und/oder Weinstein verwendet werden, die bei der Weinherstellung anfallen. Vorzugsweise beträgt der KHT-Gehalt des Rohmaterials, bezogen auf Trockensubstanz, mindestens 10 Gew.-%.

Der Erfindung liegt die Aufgabe zugrunde, eine gereinigte Weinsäure, die auch für Lebensmittel geeignet sein kann, auf umweltfreundliche und kostengünstige Weise herzustellen. Erfindungsgemäß gelingt dies dadurch,
a) daß man das Rohmaterial in einer ersten Umsetzungsstufe mit wäßriger Kalilauge verrührt und dabei KHT praktisch vollständig zu Dikaliumtartrat (DKT) umsetzt,
b) daß man aus der ersten Umsetzungsstufe eine DKT enthaltende erste wäßrige Lösung abzieht, Verunreinigungen entfernt und die erste Lösung in einer Fällungsstufe mit zudosierter Säure bei einem pH-Wert von 2,0 bis 5,0 verrührt und eine auskristallisiertes KHT enthaltende Suspension erzeugt,
c) daß man aus der Suspension auskristallisiertes KHT abtrennt, mit Wasser wäscht und eine zu mindestens 80 Gew.-% mit KHT gesättigte zweite wäßrige Lösung erzeugt, und
d) daß man aus der zweiten wäßrigen Lösung Kalium entfernt, eine wäßrige Weinsäure-Lösung erzeugt und aus der Weinsäure-Lösung Wasser mindestens teilweise entfernt.

Weinsäure hat die Summenformel H₆C₄O₆, Kaliumhydrogentartrat ist KH₅C₄O₆ und Dikaliumtartrat ist K₂H₄C₄O₆. In der ersten Umsetzungsstufe a) wird KHT mit KOH zu DKT (K₂H₄C₄O₆) plus Wasser umgesetzt, wobei man vorzugsweise bei einem pH-Wert von 7 bis 8 arbeitet. DKT ist viel besser in Wasser löslich als KHT.

Die DKT enthaltende erste wäßrige Lösung wird in der Fällungsstufe mit zudosierter Säure verrührt, wobei man das schwer lösliche KHT bildet. Es bietet sich an, als Säure Weinsäure zu verwenden, wenn man das Einbringen von Fremdsubstanzen möglichst vermeiden will. Es ist jedoch durchaus möglich, anstelle von Weinsäure zum Beispiel mit H₂SO₄ oder HCl zu arbeiten und die Sulfat- oder Chlorid-Ionen an anderer Stelle wieder aus dem Verfahren zu entfernen.

Das Verfahren ist ebenfalls geeignet, hefehaltiges Rohmaterial zu verarbeiten, welches man der ersten Umsetzungsstufe a) aufgibt. Man zieht aus der ersten Stufe eine DKT und Hefe enthaltende erste Lösung ab und trennt den hefehaltigen Schlamm ab, zum Beispiel durch Mikrofiltration, Ultrafiltration oder mittels einer oder mehrerer Zentrifugen.

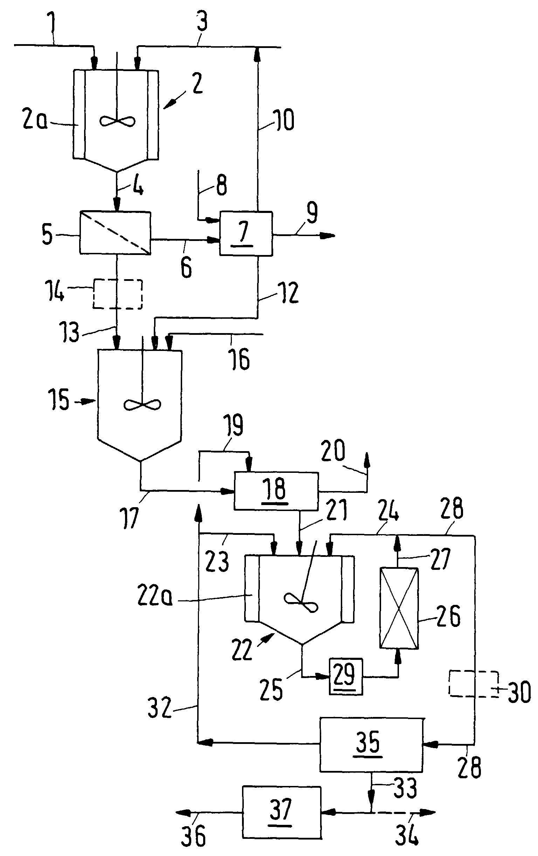

Ausgestaltungsmöglichkeiten des Verfahrens werden mit Hilfe der Zeichnung erläutert. Die Zeichnung zeigt ein Fließschema des Verfahrens.

Das KHT enthaltende Rohmaterial wird in der Leitung (1) herangeführt und einem Rührbehälter (2) aufgegeben. Im folgenden wird davon ausgegangen, daß das Rohmaterial auch hefehaltig ist. Wäßrige KOH-Lösung kommt aus der Leitung (3). Die Flüssigkeit im Behälter (2) wird zweckmäßigerweise auf etwa 60 bis 80°C beheizt, zu diesem Zweck stattet man den Behälter (2) mit einem Heizmantel (2a) aus. KOH wird in solcher Menge zudosiert, daß KHT vollständig zu DKT umgesetzt wird. Dabei liegt der pH-Wert in der Flüssigkeit des Behälters (2) üblicherweise bei 7 bis 8. Die aus dem Behälter (2) in der Leitung (4) abgezogene Lösung wird hier auch 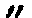 erste wäßrige Lösung" genannt. Man gibt die Lösung einer Filtration (5) auf, um einen Hefeschlamm abzutrennen, den man in der Leitung (6) abzieht und zu einer Wäsche (7) führt. Waschwasser kommt aus der Leitung (8).

In der Leitung (9) zieht man eine gewaschene Hefe ab, die zum Beispiel als Viehfutter Verwendung finden kann. Das gebrauchte Waschwasser teilt man auf und führt den ersten Teil durch die Leitung (10) zur Leitung (3) und auf diese Weise zurück in den Behälter (2). Das restliche gebrauchte Waschwasser zieht man in der Leitung (12) ab und gibt es in einen Fällungsbehälter (15).

Das Filtrat, das man aus dem Filter (5) in der Leitung (13) abzieht, enthält vor allem DKT und als Verunreinigungen unter anderem Zucker, Alkohol, verschiedene Säuren und Farbstoffe. Es kann zweckmäßig sein, diese Lösung zunächst durch eine Entfärbung (14) zu führen, die man üblicherweise als Aktivkohle-Festbett ausgestaltet. Die Entfärbung kann alternativ aber auch an einer anderen Stelle des Verfahrens erfolgen.

Dem Fällungsbehälter (15) führt man die Lösung aus der Leitung (13), das gebrauchte Waschwasser aus der Leitung (12) und eine Säure zu, welche in der Leitung (16) herangeführt wird. Bei dieser Säure kann es sich zum Beispiel um Weinsäure handeln. Die Säure wandelt das DKT praktisch vollständig in KHT um. Im Fällungsbehälter (15) sorgt man für relativ niedrige Temperaturen, vorzugsweise im Bereich von 0 bis 30°C und für einen pH-Wert von etwa 3,0 bis 5,0. Im Fällungsbehälter (15) kristallisiert KHT zumindest teilweise aus. Die gebildete kristallhaltige Suspension führt man durch die Leitung (17) zu einem Filter (18), vorzugsweise einem Bandfilter, welches mit Waschwasser beaufschlagt wird, das aus der Leitung (19) kommt. Gebrauchtes Waschwasser zieht man in der Leitung (20) ab und kann es in nicht dargestellter Weise zum Beispiel in der Hefewäsche (7) verwenden.

Die im Filter (18) gewonnenen feuchten KHT-Kristalle gibt man durch die Leitung (21) einem Rührbehälter (22) auf, der ebenfalls mit einem Heizmantel (22a) versehen ist. Im Behälter (22) löst man die Kristalle zweckmäßigerweise bei Temperaturen von 70 bis 95°C in Wasser auf, das man aus der Leitung (23) heranführt. Durch die Leitung (24) führt man dem Behälter (22) gleichzeitig Weinsäure-Lösung zu. Man zieht aus dem Rührbehälter in der Leitung (25) eine weitgehend gesättigte KHT-Lösung ab und führt sie zunächst durch eine Entfärbungsbehandlung (29), zum Beispiel ein Aktivkohlebett, und dann durch den Kationenaustauscher (26), welcher die K⁺-Ionen bindet. Auf diese Weise erzeugt man eine schon weitgehend gereinigte Weinsäure-Lösung, die neben Wasser üblicherweise aus 10 bis 20 Gew.-% Weinsäure besteht. Diese Lösung zieht man in der Leitung (27) ab, führt einen Teil in der Leitung (24) zurück in den Behälter (22) und zieht die restliche Weinsäure-Lösung durch die Leitung (28) ab. Wenn der Kationenaustauscher (26) beladen ist, wird er zum Beispiel mit Schwefelsäure oder Salzsäure regeneriert, wie es bekannt und üblich ist.

Falls eine hoch gereinigte Weinsäure angestrebt wird, führt man die Lösung der Leitung (28) noch durch einen Anionenaustauscher (30), der im Anfangszustand mit Weinsäure beladen ist. Dieser Anionenaustauscher bindet restliche Anionen, zum Beispiel Sulfat- oder Chloridionen. Die Weinsäure-Lösung fließt durch die Leitung (28) zu einer konventionellen Eindampfung (35), um Wasser zu entfernen. Das dabei gebildete wäßrige Kondensat zieht man in der Leitung (32) ab und führt es zurück durch die Leitungen (19) und (23), wie das bereits erläutert wurde. Aus der Eindampfung gewinnt man eine Weinsäure-Lösung, die üblicherweise zu 50 bis 60 Gew.-% aus Weinsäure besteht und in der Leitung (33) abgezogen wird. Ein Teilstrom dieser Lösung, den man in der Leitung (34) abzweigt, kann zur Leitung (16) zurückgeführt und in den Fällungsbehälter (15) gegeben werden. Die restliche Lösung gibt man einer ebenfalls konventionellen Kristallisation (37) auf und zieht kristallisierte Weinsäure in der Leitung (36) ab. Eine Waschung, Trocknung und Mahlung dieser Weinsäure aus der Leitung (36) kann sich anschließen, was aber zur Vereinfachung nicht dargestellt wurde.

Eine Alternative zur erläuterten Verfahrensführung besteht darin, daß man den Kationenaustauscher (26) durch eine an sich bekannte Elektrolyse ersetzt, welche die KHT-Lösung aus der Leitung (25) nach Umsetzung mit KOH zu einer DKT-Lösung elektrolytisch in eine Weinsäure-Lösung und eine KOH-Lösung aufspaltet. Die hierbei gewonnene KOH-Lösung kann zumindest teilweise zurück in den Rührbehälter (2) gegeben werden. Die gewonnene Weinsäure-Lösung fällt in der Leitung (27) an und wird in der zuvor beschriebenen Weise weiterbehandelt.

### Beispiel:

Im Labormaßstab wird Hefe aus der Weinproduktion mit einem KHT-Gehalt von 10 Gew.-% im Behälter (2) pro kg mit 2,5 kg Wasser und wäßriger KOH-Lösung (10 Gew.-% KOH) intensiv verrührt, wobei die Temperatur bei 70°C liegt. Das Gewichtsverhältnis der Zugaben an gesamtem Wasser und Hefe beträgt 2,7:1, durch die KOH-Lösung wird der pH-Wert der Suspension auf 8,0 eingestellt. Um die vollständige Umwandlung von KHT zu DKT zu erreichen, wird 1 Stunde lang gerührt.

In einer keramischen Mikrofiltration (5) trennt man bei einem Transmembrandruck von 6 bar bei 70°C aus der Suspension die Feststoffe ab, wobei ein Filterkuchen mit 30 Gew.-% Trockensubstanz entsteht. Das Permeat der Filtration, die partikelfreie DKT-Lösung, weist einen DKT-Gehalt von 10 Gew.-% auf. Dieses Permeat wird auf 15°C gekühlt und dann unter Rühren im Behälter (15) mit soviel reiner Weinsäure versetzt, bis der pH-Wert 3,5 erreicht wird. Es entsteht eine gesättigte KHT-Lösung, wobei gleichzeitig KHT kristallin ausfällt.

Die abfiltrierten KHT-Kristalle werden mehrmals mit destilliertem Wasser gewaschen und dann im Behälter (22) auf 80°C erhitzt, wobei eine gesättigte KHT-Lösung mit 44 g/l KHT entsteht. Die kristallfreie gesättigte Lösung wird über eine Aktivkohlesäule (29) geführt und dadurch entfärbt. Zur Entfernung der Kalium-Ionen leitet man die entfärbte Lösung über einen stark sauren Kationenaustauscher (26) und erhält eine gereinigte wäßrige Weinsäure-Lösung, die zur Aufkonzentrierung, Eindampfung und Kristallisation in an sich bekannter Weise geeignet ist.

## Patentansprüche

1. Verfahren zum Erzeugen von Weinsäure aus einem Rohmaterial, dessen Trockensubstanz zu mindestens 5 Gew.-% aus Kaliumhydrogentartrat (KHT) besteht, dadurch gekennzeichnet,
a) daß man das Rohmaterial in einer ersten Umsetzungsstufe mit wäßriger Kalilauge verrührt und dabei KHT praktisch vollständig zu Dikaliumtartrat (DKT) umsetzt,
b) daß man aus der ersten Umsetzungsstufe eine DKT enthaltende erste wäßrige Lösung abzieht, Verunreinigungen entfernt und die erste Lösung in einer Fällungsstufe mit zudosierter Säure bei einem pH-Wert von 2,0 bis 5,0 verrührt und eine auskristallisiertes KHT enthaltende Suspension erzeugt,
c) daß man aus der Suspension auskristallisiertes KHT abtrennt, mit Wasser wäscht und eine zu mindestens 80 Gew.-% mit KHT gesättigte zweite wäßrige Lösung erzeugt,
d) und daß man aus der zweiten wäßrigen Lösung Kalium entfernt, eine wäßrige Weinsäure-Lösung erzeugt und aus der Weinsäure-Lösung Wasser mindestens teilweise entfernt.

2. Verfahren nach Anspruch 1, dadurch gekennzeichnet, daß man der ersten Umsetzungsstufe a) ein hefehaltiges Rohmaterial aufgibt, aus der ersten Stufe eine DKT und Hefe enthaltende erste Lösung abzieht und aus der ersten Lösung einen hefehaltigen Schlamm abtrennt.

3. Verfahren nach Anspruch 1 oder 2, dadurch gekennzeichnet, daß man der Fällungsstufe Weinsäure zudosiert.

4. Verfahren nach einem der Ansprüche 1 bis 3, dadurch gekennzeichnet, daß man in der Stufe d) die zweite wäßrige Lösung durch einen Kationenaustauscher leitet, wobei K⁺-Ionen gebunden werden.

5. Verfahren nach einem der Ansprüche 1 bis 3, dadurch gekennzeichnet, daß man in der Stufe d) die zweite wäßrige Lösung mit KOH zu einer DKT-Lösung umsetzt und diese Lösung einer Elektrolyse aufgibt, aus welcher man eine Weinsäure-Lösung und, getrennt davon, eine KOH-Lösung abzieht.

6. Verfahren nach einem der Ansprüche 1 bis 5, dadurch gekennzeichnet, daß man die Weinsäure-Lösung, bevor man Wasser mindestens teilweise entfernt, durch einen im Anfangszustand mit Weinsäure beladenen Anionenaustauscher leitet.
